(19) **Europäisches Patentamt**
**European Patent Office**
**Office européen des brevets**

(11) **EP 4 714 903 A1**

(12) **EUROPEAN PATENT APPLICATION**
published in accordance with Art. 153(4) EPC

(43) Date of publication:
**25.03.2026 Bulletin 2026/13**

(21) Application number: **24807213.4**

(22) Date of filing: **15.05.2024**

(51) International Patent Classification (IPC):
*C01G 9/02* (2006.01)    *A61K 8/27* (2006.01)
*A61K 8/81* (2006.01)    *A61Q 1/00* (2006.01)
*C09D 7/61* (2018.01)

(52) Cooperative Patent Classification (CPC):
**A61K 8/27; A61K 8/81; A61Q 1/00; C01G 9/02;**
**C09D 7/61**

(86) International application number:
**PCT/JP2024/017911**

(87) International publication number:
**WO 2024/237262 (21.11.2024 Gazette 2024/47)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **15.05.2023 JP 2023080234**

(71) Applicant: **Tayca Corporation**
**Osaka-shi,**
**Osaka 551-0022 (JP)**

(72) Inventors:
• **MITO, Shunsuke**
  **Osaka-shi, Osaka 551-0022 (JP)**
• **OOSAKI, Daisuke**
  **Osaka-shi, Osaka 551-0022 (JP)**
• **MORISAKO, Yu**
  **Osaka-shi, Osaka 551-0022 (JP)**

(74) Representative: **Müller-Boré & Partner**
**Patentanwälte PartG mbB**
**Friedenheimer Brücke 21**
**80639 München (DE)**

(54) **ZINC OXIDE POWDER, METHOD FOR PRODUCING SAME, AND SURFACE-TREATED ZINC OXIDE POWDER AND COMPOSITION CONTAINING SAME**

(57)    There is provided a zinc oxide powder wherein when 5 g of the zinc oxide powder is added to 70 g of ion exchange water and the mixture is stirred, a concentration of zinc ions eluting in the water is 0.2 ppm or less. Thus, there are provided a zinc oxide powder and a surface-treated zinc oxide powder for which a concentration of zinc ions eluting in water is reduced to a specific level or less; and a composition that can reduce an environmental load and has excellent stability over time.

**EP 4 714 903 A1**

**Description**

Technical Field

The present invention relates to a zinc oxide powder and a production method therefor, and a surface-treated zinc oxide powder and a composition containing it.

Background Art

Zinc oxide has environmental toxicity due to elution of zinc ions in water, and it is known that in particular, when it is applied to an aqueous cosmetic in the field of cosmetics, eluted zinc ions interact with an anionic water-based thickener, resulting in significantly deteriorating stability of the cosmetic. Carbomer, which is a carboxyvinyl polymer, can be exemplified as an anionic water-based thickener, and when it is combined with zinc oxide, viscosity of carbomer is disadvantageously reduced due to zinc ions eluting from zinc oxide.

Patent Literature 1 has described zinc oxide coated with silicon oxide comprising zinc oxide particles and silicon oxide coating over the surface of the zinc oxide particles, wherein an aqueous suspension conductivity is 120 $\mu$S/cm or less. According to the reference, an aqueous suspension conductivity is 120 $\mu$S/cm or less, so that when the zinc oxide is applied to a hydrous material such as an aqueous cosmetic, reduction of viscosity due to carbomer can be prevented and thus quality stability of the aqueous material can be maintained. However, further inhibition of elution of zinc ions from zinc oxide has been needed.

Citation List

Patent Literature

Patent Literature 1: WO 2016/136797 A1

Summary of Invention

Technical Problem

To solve the above problems, an objective of the present invention is to provide a zinc oxide powder and a surface-treated zinc oxide powder for which a concentration of zinc ions eluting in water is reduced to a specific level or less; and a composition that can reduce an environmental load and has excellent stability over time.

Solution to Problem

The above problems are solved by providing a zinc oxide powder wherein when 5 g of the zinc oxide powder is added to 70 g of ion exchange water and the mixture is stirred, a concentration of zinc ions eluting in the water is 0.2 ppm or less.

Here, it is preferable that a BET specific surface is 0.5 to 100 m$^2$/g. It is also preferable that a ratio of a $CO_2$ desorption amount as determined by $CO_2$-TPD measurement to a BET specific surface ($CO_2$ desorption amount/BET specific surface) is 1.4 $\mu$mol/m$^2$ or more.

The above problems are also solved by providing a method for producing a zinc oxide powder, comprising a first neutralization step of adding at least one alkali source selected from the group consisting of sodium carbonate, potassium carbonate, lithium carbonate, ammonium carbonate, sodium hydrogen carbonate and ammonium hydrogen carbonate to at least one zinc source selected from the group consisting of zinc chloride, zinc sulfate, zinc acetate and zinc nitrate in a molar ratio (alkali source/zinc source) of less than 1 and then mixing the mixture; a second neutralization step of adding the alkali source such that a total molar ratio (alkali source/zinc source) is 1.05 to 5 and then mixing the mixture; a step of filtration and washing; and a step of calcination at 200 to 1000 °C.

Furthermore, the above problems are also solved by providing a surface-treated zinc oxide powder obtained by surface-treating a zinc oxide powder, wherein when 5 g of the surface-treated zinc oxide powder is added to 70 g of ion exchange water and the mixture is stirred, a concentration of zinc ions eluting in the water is 0.2 ppm or less.

Here, a preferable embodiment is a surface-treated zinc oxide powder, wherein the zinc oxide powder is surface-treated with at least one surface treating agent selected from the group consisting of a silicone oil, an aliphatic acid, an alkylsilane and a hydrous silica. It is preferable that a BET specific surface of the surface-treated zinc oxide powder is 0.5 to 70 m$^2$/g. It is preferable that a ratio of the $CO_2$ desorption amount as determined by $CO_2$-TPD measurement to a BET specific surface ($CO_2$ desorption amount/BET specific surface) is 0.2 $\mu$mol/m$^2$ or more. A preferable embodiment is a composition comprising the above surface-treated zinc oxide powder.

It is preferable that the composition further comprises a water-based thickener. It is also preferable that the water-based thickener comprises at least one selected from the group consisting of a sulfonic group, a carboxyl group and salts thereof. It is preferable that the water-based thickener is at least one copolymer selected from the group consisting of a (sodium acrylate/sodium acryloyldimethyltaurate) copolymer, a (hydroxyethyl acrylate/sodium acryloyldimethyltaurate) copolymer, an (ammonium acryloyldimethyltaurate/vinyl pyrrolidone) copolymer, a (dimethylacrylamide/sodium acryloyldimethyltaurate) crosspolymer, a carboxyvinyl polymer, xanthan gum and carrageenan. A preferable embodiment is a cosmetic composition comprising the composition. Another preferable embodiment is a paint composition comprising the composition.

Advantageous Effects of Invention

The present invention can provide a zinc oxide powder and a surface-treated zinc oxide powder for which a concentration of zinc ions eluting in water is reduced to a specific level or less. Thus, there can be provide a cosmetic or paint composition that can reduce an environmental load and has excellent stability over time.

Description of Embodiments

The zinc oxide powder of the present invention is characterized in that when 5 g of the zinc oxide powder is added to 70 g of ion exchange water and the mixture is stirred, a concentration of zinc ions eluting in the water (hereinafter, sometimes abbreviated as "zinc ion elution amount") is 0.2 ppm or less. Furthermore, the surface-treated zinc oxide powder of the present invention is a surface-treated zinc oxide powder obtained by surface-treating a zinc oxide powder, wherein when 5 g of the surface-treated zinc oxide powder is added to 70 g of ion exchange water and the mixture is stirred, a concentration of zinc ions eluting in the water (hereinafter, sometimes abbreviated as "zinc ion elution amount") is 0.2 ppm or less.

In the present invention, the condition of stirring as described above is that the mixture is mixed by shaking with a paint conditioner at 25 °C for one hour. In the present invention, a zinc ion elution amount is determined by centrifuging the solution after the stirring, filtrating a supernatant through a filter paper (5C) and a membrane filter (mesh size: 0.45 $\mu$m) and quantitating the ion elution amount in a resulting test solution with an ICP emission spectrometric apparatus.

In the zinc oxide powder and the surface-treated zinc oxide powder of the present invention, a zinc ion elution amount is 0.2 ppm or less. As seen from comparison of Examples with Comparative Examples described later, a zinc oxide powder and a surface-treated zinc oxide powder obtained in Examples have a zinc ion elution amount of 0.001 to 0.06 ppm whereas a zinc oxide powder and a surface-treated zinc oxide powder obtained in Comparative Examples have a zinc ion elution amount of 2 to 12 ppm, indicating that a zinc ion elution amount in Examples was significantly reduced compared with Comparative Examples. Then, carbomer was used in combination with a surface-treated zinc oxide powder, to prepare a composition, which was evaluated for stability over time for one month. The results show that a composition prepared with a surface-treated zinc oxide powder obtained in Examples has lower change rates of a viscosity and pH which means pH variation and viscosity reduction are effectively suppressed, that is, the composition is excellent in stability over time. Therefore, it is very significant to employ the zinc oxide powder and the surface-treated zinc oxide powder of the present invention, in which a zinc ion elution amount is reduced to a certain level or less.

If a zinc ion elution amount in the zinc oxide powder and the surface-treated zinc oxide powder of the present invention is more than 0.2 ppm, an environmental load may be insufficiently reduced, so that a composition prepared using carbomer in combination with a surface-treated zinc oxide powder has insufficient stability over time. A zinc ion elution amount is preferably 0.1 ppm or less, more preferably 0.05 ppm or less, further preferably 0.01 ppm or less, particularly preferably 0.005 ppm or less. Meanwhile, a zinc ion elution amount is generally 0.00001 ppm or more.

In the zinc oxide powder of the present invention, a BET specific surface is preferably 0.5 to 100 m$^2$/g. If a BET specific surface is less than 0.5 m$^2$/g, dispersibility may deteriorate considerably when the zinc oxide powder is applied to cosmetics. A BET specific surface is more preferably 3 m$^2$/g or more, further preferably 5 m$^2$/g or more, particularly preferably 10 m$^2$/g or more, most preferably 15 m$^2$/g or more. Meanwhile, if a BET specific surface is more than 100 m$^2$/g, ultraviolet ray shielding effect may be significantly deteriorated when the zinc oxide powder is applied to cosmetics. A BET specific surface is more preferably 90 m$^2$/g or less, further preferably 80 m$^2$/g or less, particularly preferably 70 m$^2$/g or less. A BET specific surface (m$^2$/g) in the present invention is calculated by a BET method using a fully automatic specific surface measuring device.

A CO$_2$ desorption amount of the zinc oxide powder of the present invention is preferably 15 to 200 $\mu$mol/g as determined by CO$_2$-TPD measurement. The inventors have found that the desorption amount correlates with a BET specific surface. In this light, a preferable embodiment is that a ratio of a CO$_2$ desorption amount as determined by CO$_2$-TPD measurement to a BET specific surface (CO$_2$ desorption amount/BET specific surface) is 1.4 $\mu$mol/m$^2$ or more. If the ratio (CO$_2$ desorption amount/BET specific surface) is less than 1.4 $\mu$mol/m$^2$, it becomes difficult to reduce the zinc ion elution amount. Thus, the ratio is more preferably 1.6 $\mu$mol/m$^2$ or more, further preferably 1.8 $\mu$mol/m$^2$ or more, particularly preferably 2.0 $\mu$mol/m$^2$ or more. Meanwhile, it is also preferable that the ratio ($CO_2$ desorption amount/BET specific surface) is 10 $\mu$mol/m$^2$ or less.

The surface-treated zinc oxide powder of the present invention is obtained by surface-treating a zinc oxide powder. A preferable embodiment is that the zinc oxide powder is surface-treated with at least one surface treating agent selected from the group consisting of a silicone oil, an aliphatic acid, an alkylsilane and a hydrous silica. In the present invention, "surface-treating zinc oxide powder" means that a surface treating agent is present such that it substantially covers the zinc oxide surface and thus there may be some uncoated part.

Examples of a silicone oil which can be preferably used include methicone, dimethyl polysiloxane (dimethicone), methylphenyl polysiloxane, methylhydrogen polysiloxane (hydrogen dimethicone), trimethylsiloxysilicate, and triethoxysilylethylpolydimethylsiloxyethylhexyl dimethicone. Examples of an aliphatic acid which can be preferably used include stearic acid, isostearic acid, lauric acid, myristic acid, palmitic acid and behenic acid. Examples of an alkylsilane which can be preferably used include methyltrimethoxysilane, dimethyldimethoxysilane, phenyltrimethoxysilane, methyltriethoxysilane, dimethyldiethoxysilane, triethoxycaprylylsilane, tetramethoxysilane, tetraethoxysilane, phenyltriethoxysilane, n-propyltrimethoxysilane, n-propyltriethoxysilane, hexyltrimethoxysilane, hexyltriethoxysilane, octyltriethoxysilane (triethoxycaprylylsilane), and decyltrimethoxysilane. Examples of a hydrous silica which can be preferably used include hydrolyzates of tetramethoxysilane, tetraethoxysilane or the like; methylsilicate oligomer, and ethylsilicate oligomer. These surface treating agents can be used alone or in combination of two or more.

In the surface-treated zinc oxide powder of the present invention, a BET specific surface is preferably 0.5 to 70 m$^2$/g. If a BET specific surface is less than 0.5 m$^2$/g, dispersibility may be significantly deteriorated when the zinc oxide powder is applied to cosmetics. A BET specific surface is more preferably 3 m$^2$/g or more, further preferably 5 m$^2$/g or more, particularly preferably 8 m$^2$/g or more. Meanwhile, if a BET specific surface is more than 70 m$^2$/g, ultraviolet ray shielding effect may be significantly deteriorated when the zinc oxide powder is applied to cosmetics. A BET specific surface is more preferably 60 m$^2$/g or less, further preferably 50 m$^2$/g or less. A BET specific surface (m$^2$/g) in the present invention is calculated by a BET method using a fully automatic specific surface measuring device.

In the surface-treated zinc oxide powder of the present invention, a $CO_2$ desorption amount as determined by $CO_2$-TPD measurement is preferably 4.0 to 50 $\mu$mol/g. The inventors have found that the peak area value correlates with a BET specific surface. In this light, a preferable embodiment is that a ratio of a $CO_2$ desorption amount as determined by $CO_2$-TPD measurement to a BET specific surface ($CO_2$ desorption amount/BET specific surface) is preferably 0.2 $\mu$mol/m$^2$ or more. If the ratio ($CO_2$ desorption amount/BET specific surface) is less than 0.2 $\mu$mol/m$^2$, it becomes difficult to reduce the zinc ion elution amount. It is more preferably 0.3 $\mu$mol/m$^2$ or more, further preferably 0.4 $\mu$mol/m$^2$ or more, particularly preferably 0.5 $\mu$mol/m$^2$ or more. Meanwhile, the ratio ($CO_2$ desorption amount/BET specific surface) is preferably 2.0 $\mu$mol/m$^2$ or less.

There are no particular restrictions to a production method of the present invention. The zinc oxide powder of the present invention can be suitably provided by conducting a first neutralization step of adding at least one alkali source selected from the group consisting of sodium carbonate, potassium carbonate, lithium carbonate, ammonium carbonate, sodium hydrogen carbonate and ammonium hydrogen carbonate to at least one zinc source selected from the group consisting of zinc chloride, zinc sulfate, zinc acetate and zinc nitrate in a molar ratio (alkali source/zinc source) of less than 1 and then mixing the mixture; a second neutralization step of adding the alkali source such that a total molar ratio (alkali source/zinc source) is 1.05 to 5 and then mixing the mixture; a step of filtration and washing; and a step of calcination at 200 to 1000 °C. Then, the surface-treated zinc oxide powder of the present invention can be suitably provided by surface-treating the zinc oxide powder.

As seen from comparison of Examples with Comparative Examples described later, a zinc oxide powder and a surface-treated zinc oxide powder obtained in Examples have a zinc ion elution amount of 0.001 to 0.06 ppm, which indicates that a zinc ion elution amount in Examples was significantly reduced. Meanwhile, in Comparative Examples 1 to 5 of not executing the second neutralization step, a zinc ion elution amount was 9 to 12 ppm and in Comparative Example 6 to 9 of not executing the second neutralization step in which a molar ratio (alkali source/zinc source) was 1.1 to 2 in the first neutralization step, a zinc ion elution amount was 8 to 9 ppm, indicating that reduction of a zinc ion elution amount was not insufficient. Therefore, a preferable embodiment is a production method of the present invention, comprising the first neutralization step and the second neutralization step.

In terms of the first neutralization step, a preferable method comprises adding the alkali source to the zinc source in a molar ratio (alkali source/zinc source) of less than 1 and the mixing the mixture. Preferably, both zinc source and alkali source are added as an aqueous solution. There are no particular restrictions to a time and a way for adding the alkali source to the zinc source, and the alkali source can be added at once before mixing; the alkali source can be added over a set time of 10 sec to 30 min with mixing; or the zinc source and the alkali source can be concurrently added to a reaction vessel with mixing. A reaction temperature in the first neutralization step is preferably, but not limited to, 5 to 80 °C.

In terms of the first neutralization step, if the molar ratio (alkali source/zinc source) is 1 or more, a zinc oxide powder in which reduction of a zinc ion elution amount is insufficient may be provided, and thus the molar ratio (alkali source/zinc source) is more preferably 0.98 or less, further preferably 0.96 or less, particularly preferably 0.94 or less, most preferably 0.92 or less. Meanwhile, the molar ratio (alkali source/zinc source) is preferably 0.6 or more.

A preferable embodiment is that after the first neutralization step, the second neutralization step of adding the alkali source such that a total molar ratio (alkali source/zinc source) is 1.05 to 5 and then mixing the mixture. The alkali source is preferably added as an aqueous solution and then the mixture is mixed. In terms of the second neutralization step, a preferable embodiment is that the alkali source is not added at once, but is gradually added over 30 sec to 60 min such that a total molar ratio (alkali source/zinc source) is to be 1.05 to 5 with mixing. A time for gradual addition is more preferably 2 min to 45 min, further preferably 3 min to 40 min, particularly preferably 5 min to 35 min. Alternatively, the product of the first neutralization step and the alkali source can be concurrently added to a reaction vessel with mixing. A reaction temperature in the second neutralization step is preferably, but not limited to, 5 to 80 °C.

The total molar ratio (alkali source/zinc source) in the second neutralization step means a molar ratio of the sum of the alkali sources added in the first and the second neutralization steps to the zinc source in the first neutralization step. In the second neutralization step, if the total molar ratio (alkali source/zinc source) is less than 1.05, a zinc oxide powder in which reduction of a zinc ion elution amount is insufficient may be provided, and thus, the total molar ratio (alkali source/zinc source) is more preferably 1.06 or more, further preferably 1.08 or more. Meanwhile, if the total molar ratio (alkali source/zinc source) is more than 5, ultraviolet ray shielding effect may be significantly deteriorated when the zinc oxide powder is applied to cosmetics. Thus, the total molar ratio (alkali source/zinc source) is more preferably 4 or less, further preferably 3.5 or less, particularly preferably 3 or less, most preferably 2.5 or less. Here, after the first neutralization step, filtration, washing step and then drying step can be executed.

In a preferable embodiment, after the second neutralization step, a step of filtration and washing is executed. The step of filtration and washing allows impurity such as unreacted materials to be removed. Filtration and washing are executed by a well-known method. In particular, a washing method with water is suitably employed and a preferable embodiment is that washing with water is executed until an electric conductivity becomes 500 $\mu$S/cm or less, and in a more preferable embodiment, 300 $\mu$S/cm or less.

In a preferable embodiment, after the step of filtration and washing, a drying step is executed. This advantageously allows for improving quality stability such as a specific surface in a zinc oxide powder obtained. There are no particular restrictions to a drying step, and it is preferable that after the step of filtration and washing, a cake obtained is dried by at least one method selected from the group consisting of blast drying, flash drying, spray drying, drum drying and vacuum drying. A drying temperature is preferably 80 to 150 °C. A drying time is preferably 30 min to 48 hours.

Then, in a preferable embodiment, a calcination step is executed at 200 to 1000 °C. If a calcination temperature is lower than 200 °C, purify of zinc oxide in a zinc oxide powder may be significantly lowered, and thus the temperature is more preferably 220 °C or higher, further preferably 240 °C or higher, particularly preferably 300 °C or higher. Meanwhile, if a calcination temperature is higher than 1000 °C, a specific surface may be reduced, and thus the temperature is more preferably 950 °C or lower, further preferably 900 °C or lower, particularly preferably 880 °C or lower.

A calcination time in the calcination step is preferably 30 min to 48 hours. If a calcination time is less than 1 hour, unreacted starting materials may remain, so that the zinc oxide powder of the present invention may not be obtained. A calcination time is more preferably 1 hour or more, further preferably 2 hours. Meanwhile, if a calcination time is more than 48 hours, productivity may be deteriorated. A calcination time is more preferably 24 hours or less, further preferably 15 hours or less, particularly preferably 8 hours or less.

The calcination step allows for suitably providing the zinc oxide powder of the present invention, and executing a pulverization after the calcination step is a preferable embodiment. It beneficially allows for inhibiting contamination with crude particles to provide a zinc oxide powder with higher quality stability, and also beneficially provides a uniform surface-treated zinc oxide powder when the zinc oxide powder is surface-treated. Pulverization can be executed by a well-known pulverizer or the like. Examples of a pulverizer which can be used include, but not limited to, a pin mill, a ball mill, a jet mill, a mortar mill and an ultracentrifugal pulverizer.

Then, the zinc oxide powder of the present invention can be surface-treated to provide a surface-treated zinc oxide powder. The surface-treated zinc oxide powder can be produced by a method wherein the zinc oxide powder is mixed with the surface treating agent, and it can be surface-treated by dry mixing or wet mixing using a dispersion media. Examples of a preferable dispersion media include alcohols such as methanol, ethanol and isopropanol, and toluene. In mixing during surface-treatment, preferably 0.5 to 20 parts by mass of the surface treating agent is added based on 100 parts by mass of the zinc oxide powder, and more preferably 1 to 15 parts by mass is added. Then, after optional drying and/or pulverization, the surface-treated zinc oxide powder of the present invention can be provided.

The surface-treated zinc oxide powder of the present invention can be used as a composition in combination with various components. The composition can be preferably used as a powder composition, an aqueous composition, an oil-based composition, or an emulsion composition (oil-in-water type (O/W type) or water-in-oil type (W/O type)). Among these, more preferable embodiment is a cosmetic composition comprising the surface-treated zinc oxide powder or a paint composition comprising the surface-treated zinc oxide powder. A preferable cosmetic composition containing the surface-treated zinc oxide powder of the present invention is a powder composition, an oil-based composition, or an emulsion composition (oil-in-water type (O/W type) or water-in-oil type (W/O type)). When a cosmetic composition containing the surface-treated zinc oxide powder of the present invention is an emulsion composition type (oil-in-water type (O/W type) or water-in-oil type (W/O type)), the surface-treated zinc oxide powder of the present invention is added to an aqueous or oil phase containing various cosmetic starting materials to prepare an emulsion composition, or the aqueous phase and the oil phase can be mixed to form an emulsion, to which the surface-treated zinc oxide powder of the present invention is then added to prepare an emulsion composition. The amount of the surface-treated zinc oxide powder in the composition is preferably 5 to 30% by mass.

There are no particular restrictions to various components contained in the cosmetic composition; for example, water; a polyol; a water-based thickener; an ester oil; a fat; a carbohydrate oil; or a silicone oil can be contained. Preferable examples of a polyol include glycerin, 1,3-butylene glycol, propylene glycol and polyethylene glycol. A water-based thickener can be those having at least one selected from the group consisting of (a sulfonic group, a carboxyl group, and salts thereof); preferably are used at least one copolymer selected from the group consisting of (sodium acrylate/sodium acryloyldimethyltaurate)copolymer, (hydroxyethyl acrylate/sodium acryloyldimethyltaurate)copolymer, (ammonium acryloyldimethyltaurate/vinyl pyrrolidone)copolymer, (dimethylacrylamide/sodium acryloyldimethyltaurate)crosspolymer, a carboxyvinyl polymer, xanthan gum and carrageenan. Among these, a carboxyvinyl polymer can be more preferably used as a water-based thickener. Examples of an ester oil include isopropyl myristate, cetyl isooctanoate, glyceryl trioctanoate, isononyl isononanoate, and ethylhexyl methoxycinnamate. Examples of fats include camellia oil, evening primrose oil, macadamia nut oil, olive oil, rapeseed oil, corn oil, and sesame oil. Examples of hydrocarbon oils include liquid paraffin, squalane, isoparaffin, branched-chain light paraffin, petrolatum, and ceresin. Examples of a silicone oil include dimethicone, methylphenylpolysiloxane, decamethylcyclopentasiloxane, caprylyl methicone, and methyl trimethicone. If necessary, surfactants, moisturizers, pH adjusters, nutrients, antioxidants, and fragrances can be also included.

As seen from comparison between Examples and Comparative Examples described below, compositions containing a combination of carbomer and a surface-treated zinc oxide powder were prepared and their stability over time was evaluated for one month. As a result, the compositions containing the surface-treated zinc oxide powder of the present invention had small viscosity and pH variation, effectively suppressed pH fluctuation and viscosity reduction, and exhibited excellent stability over time. Therefore, they can be suitably used as cosmetics. In particular, they can be preferably used as cosmetics for sunscreens, emulsions, creams, foundations, lipsticks, and eye shadows, and are even more preferably used as cosmetics for sunscreens and foundations.

Examples

The present invention will be explained in more detail below with reference to Examples.

[Preparation of zinc oxide powder and surface-treated zinc oxide powder]

Example 1

To 132 g of a 47 wt% aqueous zinc chloride solution prepared using zinc chloride (from FUJIFILM Wako Pure Chemical Corporation) was added 434 g of a 10 wt% aqueous sodium carbonate solution, and then gradually added 195 g of a 10 wt% aqueous sodium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 250 °C for 10 hours. The powder obtained was pulverized by a pin mill to give the zinc oxide powder of Example 1.

Example 2

To 132 g of a 47 wt% aqueous zinc chloride solution prepared using zinc chloride (from FUJIFILM Wako Pure Chemical Corporation) was added 434 g of a 10 wt% aqueous sodium carbonate solution, and then gradually added 195 g of a 10 wt% aqueous sodium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 380 °C for 3 hours. The powder obtained was pulverized by a pin mill to give the zinc oxide powder of Example 2.

Example 3

To 132 g of a 47 wt% aqueous zinc chloride solution prepared using zinc chloride (from FUJIFILM Wako Pure Chemical Corporation) was added 434 g of a 10 wt% aqueous sodium carbonate solution, and then gradually added 195 g of a 10 wt% aqueous sodium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Example 3.

Example 4

To 132 g of a 47 wt% aqueous zinc chloride solution prepared using zinc chloride (from FUJIFILM Wako Pure Chemical Corporation) was added 434 g of a 10 wt% aqueous sodium carbonate solution, and then gradually added 195 g of a 10 wt% aqueous sodium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 600 °C for 3 hours. The powder obtained was pulverized by a pin mill to give the zinc oxide powder of Example 4.

Example 5

To 132 g of a 47 wt% aqueous zinc chloride solution prepared using zinc chloride (from FUJIFILM Wako Pure Chemical Corporation) was added 434 g of a 10 wt% aqueous sodium carbonate solution, and then gradually added 195 g of a 10 wt% aqueous sodium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 850 °C for 3 hours. The powder obtained was pulverized by a pin mill to give the zinc oxide powder of Example 5.

Example 6

To 132 g of a 47 wt% aqueous zinc chloride solution prepared using zinc chloride (from FUJIFILM Wako Pure Chemical Corporation) was added 566 g of a 10 wt% aqueous potassium carbonate solution, and then gradually added 254 g of a 10 wt% aqueous potassium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Example 6.

Example 7

To 263 g of a 28 wt% aqueous zinc sulfate solution prepared using zinc sulfate heptahydrate (from FUJIFILM Wako Pure Chemical Corporation) was added 434 g of a 10 wt% aqueous sodium carbonate solution, and then gradually added 195 g of a 10 wt% aqueous sodium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Example 7.

Example 8

To 279 g of a 30 wt% aqueous zinc acetate solution prepared using zinc acetate dihydrate (from FUJIFILM Wako Pure Chemical Corporation) was added 434 g of a 10 wt% aqueous sodium carbonate solution, and then gradually added 195 g of a 10 wt% aqueous sodium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Example 8.

Example 9

To 288 g of a 30 wt% aqueous zinc nitrate solution prepared using zinc nitrate hexahydrate (from FUJIFILM Wako Pure Chemical Corporation) was added 434 g of a 10 wt% aqueous sodium carbonate solution, and then gradually added 195 g of a 10 wt% aqueous sodium carbonate solution over 10 min, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Example 9.

Example 10

To 132 g of a 47 wt% aqueous zinc chloride solution prepared using zinc chloride (from FUJIFILM Wako Pure Chemical Corporation) was added 434 g of a 10 wt% aqueous sodium carbonate solution, and then gradually added 97 g of a 10 wt% aqueous sodium carbonate solution over 10 min, to obtain a white slurry. The slurry obtained was filtrated and washed with ion exchange water until an EC of the filtrate became 150 µS/cm or less. The cake obtained was dried at 130 °C for 12 hours and then calcined at 480 °C for 3 hours. The powder obtained was pulverized by a pin mill to give the zinc oxide powder of Example 10.

Example 11

To 132 g of a 47 wt% aqueous zinc chloride solution prepared using zinc chloride (from FUJIFILM Wako Pure Chemical Corporation) was added 434 g of a 10 wt% aqueous sodium carbonate solution, and then gradually added 338 g of a 10 wt% aqueous sodium carbonate solution over 10 min, to obtain a white slurry. The slurry obtained was filtrated and washed with ion exchange water until an EC of the filtrate became 150 µS/cm or less. The cake obtained was dried at 130 °C for 12 hours and then calcined at 480 °C for 3 hours. The powder obtained was pulverized by a pin mill to give the zinc oxide powder of Example 11.

Example 12

To 132 g of a 47 wt% aqueous zinc chloride solution prepared using zinc chloride (from FUJIFILM Wako Pure Chemical Corporation) was added 434 g of a 10 wt% aqueous sodium carbonate solution, and then gradually added 531 g of a 10 wt% aqueous sodium carbonate solution over 10 min, to obtain a white slurry. The slurry obtained was filtrated and washed with ion exchange water until an EC of the filtrate became 150 µS/cm or less. The cake obtained was dried at 130 °C for 12 hours and then calcined at 480 °C for 3 hours. The powder obtained was pulverized by a pin mill to give the zinc oxide powder of Example 12.

Example 13

One hundred grams of the zinc oxide powder prepared in Example 3 was blended with 3.5 g of isostearic acid (from Kokyu Alcohol Kogyo Co., Ltd.: isostearic acid EX), and the mixture was stirred with a portable blender for 20 min. After the treatment, a powder obtained was dried at 100 °C for 2 hours, and pulverized by a pin mill, to give the surface-treated zinc oxide powder of Example 13.

Example 14

One hundred grams of the zinc oxide powder prepared in Example 3 was blended with 3.5 g of stearic acid (from Kao Corporation: purified stearic acid 450V), and the mixture was stirred with a portable blender while heating at 80 °C for 20 min. After the treatment, a powder obtained was dried at 80 °C for 2 hours, and pulverized by a pin mill, to give the surface-treated zinc oxide powder of Example 14.

Example 15

One hundred grams of the zinc oxide powder prepared in Example 3 was blended with 3.5 g of dimethicone (from Shin-Etsu Chemical Co., Ltd.: KF-96-1000cs), and the mixture was stirred with a portable blender for 20 min. After the treatment, a powder obtained was dried at 170 °C for 2 hours, and pulverized by a pin mill, to give the surface-treated zinc oxide powder of Example 15.

Example 16

One hundred grams of the zinc oxide powder prepared in Example 3 was blended with 3.5 g of hydrogen dimethicone (from Shin-Etsu Chemical Co., Ltd.: KF-9901), and the mixture was stirred with a portable blender for 20 min. After the treatment, a powder obtained was dried at 170 °C for 2 hours, and pulverized by a pin mill, to give the surface-treated zinc oxide powder of Example 16.

Example 17

One hundred grams of the zinc oxide powder prepared in Example 3 was blended with 3.5 g of triethoxycaprylylsilane (from Momentive Performance Materials Japan Limited Liability Company: SILQUEST A-137), and the mixture was stirred with a portable blender for 20 min. After the treatment, a powder obtained was dried at 170 °C for 2 hours, and pulverized by a pin mill, to give the surface-treated zinc oxide powder of Example 17.

Example 18

One hundred grams of the zinc oxide powder prepared in Example 3 was blended with 4.1 g of methyl silicate oligomer (from Mitsubishi Chemical Group Corporation: MKC silicate MS51), and the mixture was stirred with a portable blender while heating at 120 °C for 20 min. Then, 2.1 g of hydrogen dimethicone (from Shin-Etsu Chemical Co., Ltd.: KF-9901) and 1.1 g of triethoxysilylethylpolydimethylsiloxyethylhexyl dimethicone (from Shin-Etsu Chemical Co., Ltd.: KF-9909) were added, and the mixture was stirred with a portable blender for 20 min. After the treatment, a powder obtained was dried at 170 °C for 2 hours, and pulverized by a pin mill, to give the surface-treated zinc oxide powder of Example 18.

Example 19

One hundred grams of the zinc oxide powder prepared in Example 2 was blended with 8.5 g of triethoxycaprylylsilane (from Momentive Performance Materials Japan Limited Liability Company: SILQUEST A-137), and the mixture was stirred with a portable blender for 20 min. After the treatment, a powder obtained was dried at 170 °C for 2 hours, and pulverized by a pin mill, to give the surface-treated zinc oxide powder of Example 19.

Comparative Example 1

To 132 g of a 47 wt% aqueous zinc chloride solution prepared using zinc chloride (from FUJIFILM Wako Pure Chemical Corporation) was added 434 g of a 10 wt% aqueous sodium carbonate solution, to obtain a white slurry. The slurry obtained was filtrated and washed with ion exchange water until an EC of the filtrate became 150 $\mu$S/cm or less. The cake obtained was dried at 130 °C for 12 hours and then calcined at 250 °C for 10 hours. The powder obtained was pulverized by a pin mill to give the zinc oxide powder of Comparative

Example 1.

Comparative Example 2

To 132 g of a 47 wt% aqueous zinc chloride solution prepared using zinc chloride (from FUJIFILM Wako Pure Chemical Corporation) was added 434 g of a 10 wt% aqueous sodium carbonate solution, to obtain a white slurry. The slurry obtained was filtrated and washed with ion exchange water until an EC of the filtrate became 150 $\mu$S/cm or less. The cake obtained was dried at 130 °C for 12 hours and then calcined at 380 °C for 3 hours. The powder obtained was pulverized by a pin mill to give the zinc oxide powder of Comparative Example 2.

Comparative Example 3

To 132 g of a 47 wt% aqueous zinc chloride solution prepared using zinc chloride (from FUJIFILM Wako Pure Chemical Corporation) was added 434 g of a 10 wt% aqueous sodium carbonate solution, to obtain a white slurry. The slurry obtained was filtrated and washed with ion exchange water until an EC of the filtrate became 150 $\mu$S/cm or less. The cake obtained was dried at 130 °C for 12 hours and then calcined at 480 °C for 3 hours. The powder obtained was pulverized by a pin mill to give the zinc oxide powder of Comparative Example 3.

Comparative Example 4

To 132 g of a 47 wt% aqueous zinc chloride solution prepared using zinc chloride (from FUJIFILM Wako Pure Chemical Corporation) was added 434 g of a 10 wt% aqueous sodium carbonate solution, to obtain a white slurry. The slurry obtained was filtrated and washed with ion exchange water until an EC of the filtrate became 150 $\mu$S/cm or less. The cake obtained was dried at 130 °C for 12 hours and then calcined at 550 °C for 3 hours. The powder obtained was pulverized by a pin mill to give the zinc oxide powder of Comparative Example 4.

Comparative Example 5

To 132 g of a 47 wt% aqueous zinc chloride solution prepared using zinc chloride (from FUJIFILM Wako Pure Chemical Corporation) was added 434 g of a 10 wt% aqueous sodium carbonate solution, to obtain a white slurry. The slurry obtained was filtrated and washed with ion exchange water until an EC of the filtrate became 150 μS/cm or less. The cake obtained was dried at 130 °C for 12 hours and then calcined at 650 °C for 3 hours. The powder obtained was pulverized by a pin mill to give the zinc oxide powder of Comparative Example 5.

Comparative Example 6

To 132 g of a 47 wt% aqueous zinc chloride solution prepared using zinc chloride (from FUJIFILM Wako Pure Chemical Corporation) was added 531 g of a 10 wt% aqueous sodium carbonate solution, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Comparative

Example 6.

Comparative Example 7

To 132 g of a 47 wt% aqueous zinc chloride solution prepared using zinc chloride (from FUJIFILM Wako Pure Chemical Corporation) was added 627 g of a 10 wt% aqueous sodium carbonate solution, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Comparative Example 7.

Comparative Example 8

To 132 g of a 47 wt% aqueous zinc chloride solution prepared using zinc chloride (from FUJIFILM Wako Pure Chemical Corporation) was added 772 g of a 10 wt% aqueous sodium carbonate solution, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Comparative Example 8.

Comparative Example 9

To 132 g of a 47 wt% aqueous zinc chloride solution prepared using zinc chloride (from FUJIFILM Wako Pure Chemical Corporation) was added 965 g of a 10 wt% aqueous sodium carbonate solution, to obtain a white slurry. The slurry obtained was filtrated and washed with ion exchange water until an EC of the filtrate became 150 μS/cm or less. The cake obtained was dried at 130 °C for 12 hours and then calcined at 480 °C for 3 hours. The powder obtained was pulverized by a pin mill to give the zinc oxide powder of Comparative Example 9.

Comparative Example 10

One hundred grams of the zinc oxide powder prepared in Comparative Example 3 was blended with 3.5 g of isostearic acid (from Kokyu Alcohol Kogyo Co., Ltd.: isostearic acid EX), and the mixture was stirred with a portable blender for 20 min. After the treatment, a powder obtained was placed in an oven and dried at 100 °C for 2 hours, and pulverized by a pin mill, to give the surface-treated zinc oxide powder of Comparative Example 10.

Comparative Example 11

One hundred grams of the zinc oxide powder prepared in Comparative Example 3 was blended with 3.5 g of dimethicone (from Shin-Etsu Chemical Co., Ltd.: KF-96-1000cs), and the mixture was stirred with a portable blender for 20 min. After the treatment, a powder obtained was placed in an oven and dried at 170 °C for 2 hours, and pulverized by a pin mill, to give the surface-treated zinc oxide powder of Comparative Example 11.

Comparative Example 12

One hundred grams of the zinc oxide powder prepared in Comparative Example 3 was blended with 3.5 g of triethoxycaprylylsilane (from Momentive Performance Materials Japan Limited Liability Company: SILQUEST A-137), and the mixture was stirred with a portable blender for 20 min. After the treatment, a powder obtained was placed in an oven and dried at 170 °C for 2 hours, and pulverized by a pin mill, to give the surface-treated zinc oxide powder of Comparative Example 12.

Comparative Example 13

One hundred grams of the zinc oxide powder prepared in Comparative Example 2 was blended with 8.5 g of triethoxycaprylylsilane (from Momentive Performance Materials Japan Limited Liability Company: SILQUEST A-137), and the mixture was stirred with a portable blender for 20 min. After the treatment, a powder obtained was placed in an oven and dried at 170 °C for 2 hours, and pulverized by a pin mill, to give the surface-treated zinc oxide powder of Comparative Example 13.

(1) Measurement of BET specific surface area

A specific surface of the zinc oxide powders or the surface-treated zinc oxide powders obtained in the Examples and Comparative Examples was measured by the BET method using a fully automatic specific surface area analyzer (from MOUNTECH Co., Ltd., Macsorb HM model-1208). A degassing step was carried out under the conditions of 150°C and 20 minutes.

(2) Measurement of TPD-MS

Solid-state basicity of the zinc oxide powder or the surface-treated zinc oxide powder obtained in Examples and Comparative Examples was determined by a $CO_2$-TPD measurement using a catalyst analyzer (from MicrotracBEL Corp., BELCAT-B). First, 0.20 g of the zinc oxide powder or the surface-treated zinc oxide powder obtained in Examples and Comparative Examples was weighed and loaded into a quartz cell. Next, a temperature was increased to 400°C under He over 40 minutes, maintained at that temperature for 1 hour, and then decreased to 100°C under He over 10 minutes to remove adsorbed moisture. Further, $CO_2$ was adsorbed by flowing $CO_2$ gas at a flow rate of 20 mL/min for 30 minutes at 100°C. Then, the gas was switched to He, and while flowing at a flow rate of 30 mL/min, a temperature was increased to 500°C at a heating rate of 10°C/min to desorb $CO_2$. A calibration curve was created using $CO_2$/He balance gas (with known concentration, each concentration: 5%) as a standard gas, and the amount of $CO_2$ desorption was measured based on this calibration curve, and a peak area of the spectrum was digitalized (counted). A $CO_2$ peak was detected by a fragment of $CO_2$ (m/e=44) in a mass spectrum.

(3) Measurement of zinc ion elution amount

Five grams of the zinc oxide powder or the surface-treated zinc oxide powder obtained in Examples and Comparative Examples and 70 g of ion-exchange water at 25°C were weighed into a sealed container (Nikko Hansen, J Bottle, round, wide-mouth, 100 mL) and mixed by shaking for 1 hour in a paint conditioner (Red Devil, Paint Conditioner 1400) at 1725 rpm. After shaking, the solution was centrifuged, and the supernatant was filtered through a filter paper (Advantec Toyo Co., Ltd., No. 5C) and then through a membrane filter (Advantec Toyo Co., Ltd., 0.45 μm mesh size) to obtain a test solution. The amount of zinc ion elution in the test solution was quantified using an ICP-OES (from SPECTRO Analytical Instruments, ARCOS). A calibration curve was established using a general-purpose mixed standard solution (from SPEX, XSTC-22) for quantification.

[Preparation of composition]

Preparation Example 1

The oil phase components in Table 1 were accurately weighed and stirred in a disper mixer until the surface-treated zinc oxide powder of Example 13 was uniformly incorporated. The aqueous phase components in Table 1 were accurately weighed and stirred in a disper mixer while heating at 80°C. After the PEG-100 hydrogenated castor oil was completely dissolved, the oil phase components were added and stirring was continued until the mixture was uniform, to prepare the oil-in-water emulsion composition of Preparation Example 1.

[Table 1]

| | | Component | Amount (wt%) |
|---|---|---|---|
| Aqueous phase | | Carbomer 1 wt% aqueous solution (from Lubrisol, Carbopol® 934 Polymer, adjusted to pH6) | 40.0 |
| | | PEG-100 hydrogenated castor oil (from Nikko Chemicals Co., Ltd., NIKKOL HCO-100) | 1.5 |
| | | Polysorbate 80 (from Nikko Chemicals Co., Ltd., NIKKOL TO-10V) | 0.3 |
| | | BG (from KH Neochem Co., Ltd., 1,3-BG) | 4.0 |
| | | Water | 24.2 |
| Oil phase | | Caprylyl methicone (from Dow Chemical Japan Ltd., DOWSIL SS-3408) | 13.5 |
| | | Polyglyceryl-3 polydimethylsiloxyethyl dimethicone (from Shin-Etsu Chemical Co., Ltd., KF-6106) | 1.5 |
| | | Surface-treated zinc oxide powder of Example 13 | 15.0 |
| | | Total | 100.0 |

Preparation Example 2

The oil-in-water emulsion composition of Preparation Example 2 was prepared as described in Preparation Example 1, substituting the surface-treated zinc oxide powder of Example 17 for the surface-treated zinc oxide powder of Example 13.

Preparation Example 3

The oil-in-water emulsion composition of Preparation Example 3 was prepared as described in Preparation Example 1, substituting the surface-treated zinc oxide powder of Example 18 for the surface-treated zinc oxide powder of Example 13.

Preparation Example 4

The oil-in-water emulsion composition of Preparation Example 4 was prepared as described in Preparation Example 1, substituting the surface-treated zinc oxide powder of Example 19 for the surface-treated zinc oxide powder of Example 13.

Preparation Example 5

The oil phase components in Table 2 were accurately weighed and stirred in a disper mixer until the surface-treated zinc oxide powder of Example 19 was uniformly incorporated. The aqueous phase components in Table 2 were accurately weighed and stirred in a disper mixer while heating at 80°C. After the PEG-100 hydrogenated castor oil was completely dissolved, the oil phase components were added and stirring was continued until the mixture was uniform, to prepare the oil-in-water emulsion composition of Preparation Example 5.

[Table 2]

| | | Component | Amount (wt%) |
|---|---|---|---|
| Aqueous phase | | Carbomer 1 wt% aqueous solution (from Lubrisol, Carbopol® 934 Polymer, adjusted to pH 6) | 40.0 |
| | | PEG-100 hydrogenated castor oil (from Nikko Chemicals Co., Ltd., NIKKOL HCO-100) | 1.5 |
| | | Polysorbate 80 (from Nikko Chemicals Co., Ltd., NIKKOL TO-10V) | 0.3 |
| | | BG (from KH Neochem Co., Ltd., 1,3-BG) | 4.0 |
| | | Water | 24.2 |

(continued)

| | Component | Amount (wt%) |
|---|---|---|
| Oil phase | Caprylyl methicone (from Dow Chemical Japan Ltd., DOWSIL SS-3408) | 12.5 |
| | Polyglyceryl-3 polydimethylsiloxyethyl dimethicone (from Shin-Etsu Chemical Co., Ltd., KF-6106) | 1.5 |
| | Ethylhexyl methoxycinnamate (from BASF Japan, Uvinul® MC 80) | 1.0 |
| | Surface-treated zinc oxide powder of Example 19 | 15.0 |
| | Total | 100.0 |

Preparation Example 6

The oil phase components in Table 3 were accurately weighed and stirred in a disper mixer until the surface-treated zinc oxide powder of Example 19 and the stearic acid-treated titanium oxide were uniformly incorporated. The aqueous phase components in Table 3 were accurately weighed and stirred in a disper mixer while heating at 80°C. After the PEG-100 hydrogenated castor oil was completely dissolved, the oil phase components were added and stirring was continued until the mixture was uniform, to prepare the oil-in-water emulsion composition of Preparation Example 6.

[Table 3]

| | Component | Amount (wt%) |
|---|---|---|
| Aqueous phase | Carbomer 1 wt% aqueous solution (from Lubrisol, Carbopol® 934 Polymer, adjusted to pH 6) | 40.0 |
| | PEG-100 hydrogenated castor oil (from Nikko Chemicals Co., Ltd., NIKKOL HCO-100) | 1.5 |
| | Polysorbate 80 (from Nikko Chemicals Co., Ltd., NIKKOL TO-10V) | 0.3 |
| | BG (from KH Neochem Co., Ltd., 1,3-BG) | 4.0 |
| | Water | 24.2 |
| Oil phase | Caprylyl methicone (from Dow Chemical Japan Ltd., DOWSIL SS-3408) | 11.5 |
| | Polyglyceryl-3 polydimethylsiloxyethyl dimethicone (from Shin-Etsu Chemical Co., Ltd., KF-6106) | 1.5 |
| | Surface-treated zinc oxide powder of Example 19 | 15.0 |
| | Stearic acid-treated titanium oxide (from TAYCA Corporation, MICRO TITANIUM DIOXIDE MT-200ST) | 2.0 |
| | Total | 100.0 |

Preparation Example 7

The aqueous phase components in Table 4 were accurately weighed and mixed until uniform. The oil phase components were accurately weighed and mixed in a disper mixer until the surface-treated zinc oxide powder of Example 19 and the stearic acid-treated titanium oxide were uniformly mixed. The aqueous phase was added while stirring the oil phase in a disper mixer, and the stirring was continued until the system became uniform, to prepare the water-in-oil emulsion composition of Preparation Example 7.

[Table 4]

| | Component | Amount (wt%) |
|---|---|---|
| Oil phase | Dimethicone (from Shin-Etsu Chemical Co., Ltd., KF-96L-1.5cs) | 58.0 |
| | PEG-9 polydimethylsiloxyethyl dimethicone (from Shin-Etsu Chemical Co., Ltd., KF-6028P) | 2.0 |
| | Surface-treated zinc oxide powder of Example 19 | 15.0 |
| | Stearic acid-treated titanium oxide (from TAYCA Co., Ltd., MICRO TITANIUM DIOXIDE MT-200ST) | 5.0 |
| Aqueous phase | BG (from KH Neochem Co., Ltd., 1,3-BG) | 4.0 |
| | Water | 16.0 |
| | Total | 100.0 |

Comparative Preparation Example 1

The oil-in-water emulsion composition of Comparative Preparation Example 1 was prepared as described in Preparation Example 1, substituting the surface-treated zinc oxide powder of Comparative Example 6 for the surface-treated zinc oxide powder of Example 13.

Comparative Preparation Example 2

The oil-in-water emulsion composition of Comparative Preparation Example 2 was prepared as described in Preparation Example 1, substituting the surface-treated zinc oxide powder of Comparative Example 8 for the surface-treated zinc oxide powder of Example 13.

Comparative Preparation Example 3

The oil-in-water emulsion composition of Comparative Preparation Example 3 was prepared as described in Preparation Example 1, substituting the surface-treated zinc oxide powder of Comparative Example 12 for the surface-treated zinc oxide powder of Example 13.

Comparative Preparation Example 4

The oil-in-water emulsion composition of Comparative Preparation Example 4 was prepared as described in Preparation Example 7, substituting the surface-treated zinc oxide powder of Comparative Example 12 for the surface-treated zinc oxide powder of Example 19.

(4) Measurement of viscosity

An oil-in-water emulsion composition obtained in Preparation Examples and Comparative Preparation Examples was filled into a 30 mL screw vial, and while the vial was left to stand, viscosity was recorded 1 minute after the start of measurement at 12 rpm using a B-type viscometer (from Toki Sangyo Co., Ltd., TVB-10).

(5) Measurement of pH

An oil-in-water emulsion composition obtained in Preparation Examples and Comparative Preparation Examples was filled into a 30 mL screw vial, and pH was recorded 5 minutes after the start of measurement using a pH meter (from HORIBA, D-51S) calibrated with calibration solutions of pH 4, 7, and 9.

(6) Variation after one month at 50°C

A viscosity and a pH of an oil-in-water emulsion composition obtained in Preparation Examples and Comparative Preparation Examples were measured immediately after preparation and after storage for one month in a thermostatic bath maintained at 50°C, and then variation in a viscosity and a pH at 25°C was calculated using the following formulas. An oil-in-water emulsion composition stored at 50°C was immersed in water at 25°C for 3 hours, and a viscosity and a pH were measured after confirming that a temperature had reached 25°C.

Variation of viscosity = [(Viscosity at 25°C after 1 month at 50°C) - (Viscosity at 25°C immediately after preparation)]/ (Viscosity at 25°C immediately after preparation) Variation of pH = [(pH at 25°C after 1 month at 50°C) - (pH at 25°C immediately after preparation)]/(pH at 25°C immediately after preparation)

(7) Measurement of the amount of zinc ions eluted from a water-in-oil emulsion composition

A water-in-oil emulsion compositions obtained in Preparation Examples and Comparative Preparation Examples was applied to a PMMA plate (from HelioScreen, HERIOPLATE HD6) at 1.3 mg/cm$^2$ in accordance with ISO 24443 and allowed to dry in a dark place at 25°C for 30 minutes. Subsequently, 300 g of ion exchange water was weighed into a 500 mL beaker (from HARIO, B-500 SCI). While stirring with a stirrer and a stirring bar, the PMMA plate coated with the water-in-oil emulsion composition was immersed in ion-exchange water such that the entire surface was immersed. After 24 hours, the ion exchange water was filtered through a membrane filter (from ADVANTEC, 0.25 $\mu$m mesh size), and the amount of zinc ions eluted was quantified using an ICP-OES (from, SPECTRO Analytical Instruments GmbH, ARCOS). For quantification, a calibration curve was established using general-purpose mixed standard solutions (from SPEX, XSTC-22).

[Table 5]

| | | Starting materials | | | | | | | | | | | | Calcination conditions | | Physical properties (untreated material, substrate) | | | | |
|---|---|---|---|---|---|---|---|---|---|---|---|---|---|---|---|---|---|---|---|---|
| | Zinc source | Zinc source concentration (wt%) | Amount (g) | Zinc source material amount (mmol) | Alkali source | Alkali source concentration (wt%) | Alkali source Amount (1) (g) | Alkali source material amount (mmol) | Alkali source /zinc molar ratio | Alkali source Amount (2) (g) | Added material amount (mmol) | Alkali /zinc molar ratio | Total alkali /zinc molar ratio | Temperature (°C) | Time (h) | Specific surface (m²/g) | TPD ($CO_2$) peak area (count) | $CO_2$ desorption amount (µmol/g) | $CO_2$ desorption amount /Specific surface (µmol/m²) | Ion elution amount (ppm) |
| Example 1 | Zinc chloride | 47 | 132 | 45.5 | Sodium carbonate | 10 | 434 | 40.9 | 0.90 | 195 | 18.4 | 0.40 | 1.30 | 250 | 10 | 69 | 311.908 | 118.5 | 1.72 | 0.001 |
| Example 2 | Zinc chloride | 47 | 132 | 45.5 | Sodium carbonate | 10 | 434 | 40.9 | 0.90 | 195 | 18.4 | 0.40 | 1.30 | 380 | 3 | 51 | 233.553 | 88.8 | 1.74 | 0.001 |
| Example 3 | Zinc chloride | 47 | 132 | 45.5 | Sodium carbonate | 10 | 434 | 40.9 | 0.90 | 195 | 18.4 | 0.40 | 1.30 | 480 | 3 | 20 | 141.591 | 53.8 | 2.69 | 0.001 |
| Example 4 | Zinc chloride | 47 | 132 | 45.5 | Sodium carbonate | 10 | 434 | 40.9 | 0.90 | 195 | 18.4 | 0.40 | 1.30 | 600 | 3 | 11 | 80.653 | 30.6 | 2.79 | 0.001 |
| Example 5 | Zinc chloride | 47 | 132 | 45.5 | Sodium carbonate | 10 | 434 | 40.9 | 0.90 | 195 | 18.4 | 0.40 | 1.30 | 850 | 3 | 4 | 45.623 | 17.3 | 4.33 | 0.001 |
| Example 6 | Zinc chloride | 47 | 132 | 45.5 | Potassium carbonate | 10 | 566 | 41.0 | 0.90 | 254 | 18.4 | 0.40 | 1.30 | 480 | 3 | 21 | 111.28 | 42.3 | 2.01 | 0.001 |
| Example 7 | Zinc sulfate | 28 | 263 | 45.6 | Sodium carbonate | 10 | 434 | 40.9 | 0.90 | 195 | 18.4 | 0.40 | 1.30 | 480 | 3 | 20 | 90.921 | 34.5 | 1.73 | 0.001 |
| Example 8 | Zinc acetate | 30 | 279 | 45.6 | Sodium carbonate | 10 | 434 | 40.9 | 0.90 | 195 | 18.4 | 0.40 | 1.30 | 480 | 3 | 22 | 95.218 | 36.2 | 1.64 | 0.001 |
| Example 9 | Zinc nitrate | 30 | 288 | 45.6 | Sodium carbonate | 10 | 434 | 40.9 | 0.90 | 195 | 18.4 | 0.40 | 1.30 | 480 | 3 | 20 | 88.536 | 33.6 | 1.68 | 0.001 |
| Example 10 | Zinc chloride | 47 | 132 | 45.5 | Sodium carbonate | 10 | 434 | 40.9 | 0.90 | 97 | 9.15 | 0.20 | 1.10 | 480 | 3 | 20 | 148.354 | 56.4 | 2.82 | 0.001 |
| Example 11 | Zinc chloride | 47 | 132 | 45.5 | Sodium carbonate | 10 | 434 | 40.9 | 0.90 | 338 | 31.9 | 1.60 | 1.60 | 480 | 3 | 21 | 152.728 | 58.0 | 2.76 | 0.001 |
| Example 12 | Zinc chloride | 47 | 132 | 45.5 | Sodium carbonate | 10 | 434 | 40.9 | 0.90 | 531 | 50.1 | 2.00 | 2.00 | 480 | 3 | 22 | 165.827 | 63.0 | 2.86 | 0.001 |

[Table 5] continued

| | | Starting materials | | | | | | | | | | | | Calcination conditions | | Physical properties (untreated material, substrate) | | | | |
|---|---|---|---|---|---|---|---|---|---|---|---|---|---|---|---|---|---|---|---|---|
| | Zinc source | Zinc source concentration (wt%) | Amount (g) | Zinc source material amount (mmol) | Alkali source | Alkali source concentration (wt%) | Alkali source Amount (1) (g) | Alkali source material amount (mmol) | Alkali source /zinc molar ratio | Alkali source Amount (2) (g) | Added material amount (mmol) | Alkali /zinc molar ratio | Total alkali /zinc molar ratio | Temperature (°C) | Time (h) | Specific surface (m²/g) | TPD ($CO_2$) peak area (count) | $CO_2$ desorption amount (μmol/g) | $CO_2$ desorption amount /Specific surface (μmol/m²) | Ion elution amount (ppm) |
| Comparative Example 1 | Zinc chloride | 47 | 132 | 45.5 | Sodium carbonate | 10 | 434 | 40.9 | 0.90 | - | - | - | - | 250 | 10 | 64 | 102.988 | 39.1 | 0.61 | 12 |
| Comparative Example 2 | Zinc chloride | 47 | 132 | 45.5 | Sodium carbonate | 10 | 434 | 40.9 | 0.90 | - | - | - | - | 380 | 3 | 46 | 79.823 | 30.3 | 0.66 | 12 |
| Comparative Example 3 | Zinc chloride | 47 | 132 | 45.5 | Sodium carbonate | 10 | 434 | 40.9 | 0.90 | - | - | - | - | 480 | 3 | 18 | 33.568 | 12.8 | 0.71 | 11 |
| Comparative Example 4 | Zinc chloride | 47 | 132 | 45.5 | Sodium carbonate | 10 | 434 | 40.9 | 0.90 | - | - | - | - | 550 | 3 | 9 | 16.345 | 6.2 | 0.69 | 9 |
| Comparative Example 5 | Zinc chloride | 47 | 132 | 45.5 | Sodium carbonate | 10 | 434 | 40.9 | 0.90 | - | - | - | - | 650 | 3 | 4 | 8.754 | 3.3 | 0.83 | 9 |
| Comparative Example 6 | Zinc chloride | 47 | 132 | 45.5 | Sodium carbonate | 10 | 531 | 50.1 | 1.10 | - | - | - | - | 480 | 3 | 18 | 55.119 | 20.9 | 1.16 | 8 |
| Comparative Example 7 | Zinc chloride | 47 | 132 | 45.5 | Sodium carbonate | 10 | 627 | 59.2 | 1.30 | - | - | - | - | 480 | 3 | 18 | 55.673 | 21.2 | 1.18 | 8 |
| Comparative Example 8 | Zinc chloride | 47 | 132 | 45.5 | Sodium carbonate | 10 | 772 | 72.8 | 1.60 | - | - | - | - | 480 | 3 | 17 | 57.834 | 22.0 | 1.29 | 8 |
| Comparative Example 9 | Zinc chloride | 47 | 132 | 45.5 | Sodium carbonate | 10 | 965 | 91.0 | 2.00 | - | - | - | - | 480 | 3 | 17 | 53.568 | 20.4 | 1.20 | 9 |

[Table 6]

| | Starting materials | Surface treatment | | | Physical properties (Surface-treated material) | | | | |
|---|---|---|---|---|---|---|---|---|---|
| | Zinc oxide powder | Step | Surface treating agent | Treatment amount (based on a substrate, wt%) | Specific surface | TPD ($CO_2$) peak area (count) | $CO_2$ desorption amount ($\mu$mol/g) | $CO_2$ desorption amount /Specific surface ($\mu$mol/m$^2$) | Ion elution amount ppm |
| Example 13 | Zinc oxide powder of Example 3 | Dry | Isostearic acid | 3.5 | 13 | 21.378 | 8.1 | 0.6 | 0.002 |
| Example 14 | Zinc oxide powder of Example 3 | Dry | Stearic acid | 3.5 | 15 | 20.462 | 7.8 | 0.5 | 0.001 |
| Example 15 | Zinc oxide powder of Example 3 | Dry | Dimethicone | 3.5 | 18 | 24.590 | 9.3 | 0.5 | 0.002 |
| Example 16 | Zinc oxide powder of Example 3 | Dry | Hydrogen dimethicone | 3.5 | 16 | 25.409 | 9.7 | 0.6 | 0.002 |
| Example 17 | Zinc oxide powder of Example 3 | Dry | Triethoxycaprylylsilane | 3.5 | 17 | 22.154 | 8.4 | 0.5 | 0.001 |
| Example 18 | Zinc oxide powder of Example 3 | Dry | (1) Hydrous silica (2) Hydrogen dimethicone (3) Triethoxysilylethylpolydimethylsiloxyethylhexyl dimethicone | (1) 4.1 (2) 2.1 (3) 1.1 | 10 | 26.745 | 10.2 | 1.0 | 0.002 |
| Example 19 | Zinc oxide powder of Example 2 | Dry | Triethoxycaprylylsilane | 8.5 | 41 | 46.576 | 17.7 | 0.4 | 0.06 |
| Comparative Example 10 | Zinc oxide powder of Comparative Example 3 | Dry | Isostearic acid | 3.5 | 12 | 3.656 | 1.4 | 0.1 | 4 |
| Comparative Example 11 | Zinc oxide powder of Comparative Example 3 | Dry | Dimethicone | 3.5 | 16 | 4.023 | 1.5 | 0.1 | 3 |
| Comparative Example 12 | Zinc oxide powder of Comparative Example 3 | Dry | Triethoxycaprylylsilane | 3.5 | 14 | 3.983 | 1.5 | 0.1 | 3 |

(continued)

| | Starting materials | Surface treatment | | | Physical properties (Surface-treated material) | | | | |
|---|---|---|---|---|---|---|---|---|---|
| | Zinc oxide powder | Step | Surface treating agent | Treatment amount (based on a substrate, wt%) | Specific surface | TPD ($CO_2$) peak area (count) | $CO_2$ desorption amount ($\mu$mol/g) | $CO_2$ desorption amount /Specific surface ($\mu$mol/m$^2$) | Ion elution amount ppm |
| Comparative Example 13 | Zinc oxide powder of Comparative Example 2 | Dry | Triethoxycaprylylsilane | 8.5 | 38 | 6.752 | 2.6 | 0.1 | 2 |

[Table 7]

| | Sample | Dosage form of formulation | Analysis on the preparation day | | Analysis after one month at 50°C | | Variation after one month at 50°C | | Zinc ion elution amount from a water-in-oil emulsion composition ppm |
|---|---|---|---|---|---|---|---|---|---|
| | | | Viscosity (mPa·s) | pH | Viscosity (mPa·s) | pH | Viscosity (mPa·s) | pH | - |
| Preparation Example 1 | Example 13 | O/W | 49500 | 6.5 | 44500 | 7.6 | -10% | 17% | - |
| Preparation Example 2 | Example 17 | O/W | 46200 | 6.7 | 40150 | 7.8 | -13% | 16% | - |
| Preparation Example 3 | Example 18 | O/W | 47550 | 6.6 | 38250 | 7.6 | -20% | 15% | - |
| Preparation Example 4 | Example 19 | O/W | 48750 | 6.6 | 36800 | 7.5 | -25% | 14% | - |
| Preparation Example 5 | Example 19 | O/W | 41900 | 6.9 | 30150 | 7.9 | -28% | 14% | - |
| Preparation Example 6 | Example 19 | O/W | 46150 | 6.2 | 39850 | 7.1 | -14% | 15% | - |
| Preparation Example 7 | Example 19 | W/O | - | - | - | - | - | - | 0.02 |
| Comparative Preparation Example 1 | Comparative Example 6 | O/W | 25650 | 6.8 | 7250 | 8.8 | -72% | 29% | - |
| Comparative Preparation Example 2 | Comparative Example 8 | O/W | 19950 | 6.7 | 6500 | 9.2 | -67% | 37% | - |
| Comparative Preparation Example 3 | Comparative Example 12 | O/W | 28700 | 6.8 | 6800 | 9.1 | -76% | 34% | - |
| Comparative Preparation Example 4 | Comparative Example 12 | W/O | - | - | - | - | - | - | 1.3 |

**Claims**

1. A zinc oxide powder wherein when 5 g of the zinc oxide powder is added to 70 g of ion exchange water and the mixture is stirred, a concentration of zinc ions eluting in the water is 0.2 ppm or less.

2. The zinc oxide powder according to Claim 1, wherein a BET specific surface is 0.5 to 100 $m^2$/g.

3. The zinc oxide powder according to Claim 1 or 2, wherein a ratio of a $CO_2$ desorption amount as determined by $CO_2$-TPD measurement to a BET specific surface ($CO_2$ desorption amount/BET specific surface) is 1.4 $\mu$mol/$m^2$ or more.

4. A method for producing the zinc oxide powder according to any one of Claims 1 to 3, comprising a first neutralization step of adding at least one alkali source selected from the group consisting of sodium carbonate, potassium carbonate, lithium carbonate, ammonium carbonate, sodium hydrogen carbonate and ammonium hydrogen carbonate to at least one zinc source selected from the group consisting of zinc chloride, zinc sulfate, zinc acetate and zinc nitrate in a molar ratio (alkali source/zinc source) of less than 1 and then mixing the mixture; a second neutralization step of adding the alkali source such that a total molar ratio (alkali source/zinc source) is 1.05 to 5 and then mixing the mixture; a step of filtration and washing; and a step of calcination at 200 to 1000 °C.

5. A surface-treated zinc oxide powder obtained by surface-treating a zinc oxide powder, wherein when 5 g of the surface-treated zinc oxide powder is added to 70 g of ion exchange water and the mixture is stirred, a concentration of zinc ions eluting in the water is 0.2 ppm or less.

6. The surface-treated zinc oxide powder according to Claim 5, wherein the zinc oxide powder is surface-treated with at least one surface treating agent selected from the group consisting of a silicone oil, an aliphatic acid, an alkylsilane and a hydrous silica.

7. The surface-treated zinc oxide powder according to Claim 5 or 6, wherein a BET specific surface is 0.5 to 70 $m^2$/g.

8. The surface-treated zinc oxide powder according to any one of Claims 5 to 7, wherein a ratio of the $CO_2$ desorption amount as determined by $CO_2$-TPD measurement to a BET specific surface ($CO_2$ desorption amount/BET specific surface) is 0.2 $\mu$mol/$m^2$ or more.

9. A composition comprising the surface-treated zinc oxide powder according to any one of Claims 5 to 8.

10. The composition according to Claim 9, further comprising a water-based thickener.

11. The composition according to Claim 10, wherein the water-based thickener comprises at least one selected from the group consisting of a sulfonic group, a carboxyl group and salts thereof.

12. The composition according to Claim 11, wherein the water-based thickener is at least one copolymer selected from the group consisting of a (sodium acrylate/sodium acryloyldimethyltaurate) copolymer, a (hydroxyethyl acrylate/sodium acryloyldimethyltaurate) copolymer, an (ammonium acryloyldimethyltaurate/vinyl pyrrolidone) copolymer, a (dimethylacrylamide/sodium acryloyldimethyltaurate) crosspolymer, a carboxyvinyl polymer, xanthan gum and carrageenan.

13. A cosmetic composition comprising the composition according to any one of Claims 9 to 12.

14. A paint composition comprising the composition according to any one of Claims 9 to 12.

**EP 4 714 903 A1**

## INTERNATIONAL SEARCH REPORT

| International application No. |
| --- |
| **PCT/JP2024/017911** |

| A. | CLASSIFICATION OF SUBJECT MATTER |
| --- | --- |

*C01G 9/02*(2006.01)i; *A61K 8/27*(2006.01)i; *A61K 8/81*(2006.01)i; *A61Q 1/00*(2006.01)i; *C09D 7/61*(2018.01)i
FI:   C01G9/02 A; A61K8/27; A61K8/81; A61Q1/00; C01G9/02 B; C09D7/61

According to International Patent Classification (IPC) or to both national classification and IPC

| B. | FIELDS SEARCHED |
| --- | --- |

Minimum documentation searched (classification system followed by classification symbols)

C01G9/02; A61K8/27; A61K8/81; A61Q1/00; C09D7/61

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2003-292818 A (SHOWA DENKO K.K.) 15 October 2003 (2003-10-15) | 1-3, 5-11, 14 |
| | claims, paragraphs [0001], [0067]-[0076], [0083], example 1, table 3 | |
| Y | | 12-14 |
| A | | 4 |
| X | WO 2011/034032 A1 (SUMITOMO OSAKA CEMENT CO., LTD.) 24 March 2011 (2011-03-24) | 1-3, 5-13 |
| | claims, paragraph [0059], example 1, table 1 | |
| Y | | 12-14 |
| A | | 4 |
| X | JP 4849586 B2 (SHOWA DENKO K.K.) 11 January 2012 (2012-01-11) | 1-3, 5-13 |
| | claims, production example 4, table 8 | |
| Y | | 12-14 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **19 July 2024** | **30 July 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**EP 4 714 903 A1**

**INTERNATIONAL SEARCH REPORT**

| International application No. |
|---|
| **PCT/JP2024/017911** |

| C. | DOCUMENTS CONSIDERED TO BE RELEVANT | |
|---|---|---|
| Category* | Citation of document, with indication, where appropriate, of the relevant passages | Relevant to claim No. |
| A | CN 102616828 A (JIANGSU DONGTAI FINE CHEMICAL CO., LTD.) 01 August 2012 (2012-08-01) entire text | 4 |
| P, X | JP 2023-084113 A (SAKAI CHEMICAL INDUSTRY CO.) 16 June 2023 (2023-06-16) claims, paragraph [0058], examples, tables 1-2 | 1-3, 5-14 |
| P, A | | 4 |

Form PCT/ISA/210 (second sheet) (July 2022)

**INTERNATIONAL SEARCH REPORT**
Information on patent family members

International application No.

**PCT/JP2024/017911**

| Patent document cited in search report | | | Publication date (day/month/year) | Patent family member(s) | Publication date (day/month/year) |
|---|---|---|---|---|---|
| JP | 2003-292818 | A | 15 October 2003 | (Family: none) | |
| WO | 2011/034032 | A1 | 24 March 2011 | US 2012/0177707 A1<br>claims, paragraph [0122],<br>example 1, table 1<br>US 2015/0147370 A1<br>EP 2479213 A1<br>CN 102498169 A<br>KR 10-2012-0071389 A | |
| JP | 4849586 | B2 | 11 January 2012 | US 2002/0041853 A1<br>claims, production example 4,<br>table 8<br>WO 2001/093812 A1<br>claims, production example 4,<br>table 8<br>EP 1287807 A1<br>CN 1441659 A | |
| CN | 102616828 | A | 01 August 2012 | (Family: none) | |
| JP | 2023-084113 | A | 16 June 2023 | (Family: none) | |

Form PCT/ISA/210 (patent family annex) (July 2022)

24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016136797 A1